# EUROPEAN PATENT APPLICATION

(11) **EP 4 831 198 A1**
(43) Date of publication of application: **28.10.2026**
(21) Application number: 23962339.0
(22) Date of filing: 21.12.2023
(51) Int. Cl.: C07K 16/28, A61P 35/00, A61K 39/00

(54) **ANTIBODY SPECIFICALLY BINDING TO CD73 AND USE THEREOF**

(71) Applicant: GI Innovation, Inc., Seoul 05855 (KR)
(72) Inventor: JANG, Myung Ho, Seoul 05855 (KR); KOH, Young Jun, Seoul 05855 (KR); SHIN, Kayoung, Seoul 05855 (KR)
(74) Representative: HGF
(86) International application number: PCT/KR2023/021193
(87) International publication number: WO 2025/135233

(57) **Abstract**

The present invention relates to an antibody or an antigen-binding fragment thereof that specifically binds to CD73 protein, and a pharmaceutical composition for preventing or treating cancer comprising the same. The antibody of the present invention binds not only to human CD73 but also to non-human CD73, for example, cynomolgus monkey CD73 and mouse CD73, can be stably produced with high purity, and exhibits CD73-binding ability and inhibitory activity equivalent or superior to those of Oleclumab, a commercially available anti-CD73 antibody. In particular, the anti-CD73 single-domain antibody of the present invention exhibits approximately 1.5- to 5-fold greater CD73 enzymatic inhibitory activity compared to Oleclumab, thus may function as an excellent alternative therapeutic agent for patients with low responsiveness to Oleclumab.

## Description

### Technical Field

The present invention relates to a novel antibody that specifically binds to CD73, or an antigen-binding fragment thereof, and to a method for the treatment or diagnosis of cancer using the same.

### Background Art

Cancer immunotherapy is a therapeutic approach for treating cancer using the body's immune system. It may induce the immune system to attack cancer cells by targeting antigens, such as proteins expressed on the surface of cancer cells, and can enhance immune cell activity by modulating the tumor microenvironment associated with immune evasion of cancer cells.

The tumor microenvironment refers to the environment in which cancer cells proliferate and progress, and includes not only cancer cells but also fibroblasts, blood vessels, lymphatic vessels, immune cells, extracellular matrix, and adipocytes present within tumor tissues. In this context, it has been reported that the ATP-AMP-adenosine-A2AR/A2BR signaling pathway regulates immune cell activity in the tumor microenvironment (S. Vigano et al., Front Immunol (2019) 10:925). Specifically, when adenosine binds to A2AR/A2BR receptors on the surface of tumor cells or immune cells, immunosuppressive cells such as regulatory T cells and tumor-associated macrophages (TAMs) are activated, while cytotoxic T cells and natural killer (NK) cells are suppressed. Accordingly, approaches for activating immune responses in the tumor microenvironment by inhibiting this signaling pathway through targeting CD39, an ectonucleotidase that converts ATP to AMP; CD73, an ectonucleotidase that converts AMP to adenosine; or A2AR/A2BR receptors that bind adenosine, have been actively investigated.

Meanwhile, CD73 is involved in the production of adenosine, is highly expressed in cancer cells, and is known to promote immunosuppressive effects. It is expressed in various cancers including leukemia, bladder cancer, glioma, glioblastoma, ovarian cancer, melanoma, prostate cancer, thyroid cancer, esophageal cancer, and breast cancer. In addition, CD73 is also known to be expressed on the surface of immunosuppressive cells including regulatory T cells (Tregs) and myeloid-derived suppressor cells (MDSCs). In particular, CD73 overexpression has been reported to be associated with angiogenesis, invasion, resistance to chemotherapy, metastasis and poor survival outcomes in various tumors, including breast cancer and melanoma.

Throughout the present specification, a number of publications and patent documents are referred to and cited. The disclosure of the cited publications and patent documents is incorporated herein by reference in its entirety to more clearly describe the state of the art to which the present invention pertains and the content of the present invention.

### DISCLOSURE

### Technical Problem

The present inventors have made intensive studies to develop an effective anti-CD73 antibody that significantly inhibits tumor survival and growth by binding with high affinity to the CD73 protein, which is highly expressed in cancer cells within the tumor microenvironment and acts as a key regulator of angiogenesis and immune evasion mechanisms, thereby provides reliable diagnostic information for tumors. As a result, the present inventors have successfully identified a number of antibodies that recognize CD73 on the surface of cancer cells with high affinity and specificity, and clearly elucidated the structure of their antigen-binding regions, thereby completing the present invention.

Accordingly, it is an object of the present invention to provide an antibody or an antigen-binding fragment thereof that specifically binds to CD73, and a nucleic acid molecule encoding the same.

It is another object of the present invention to provide a pharmaceutical composition for preventing or treating cancer comprising said antibody or antigen-binding fragment thereof as an active ingredient.

Other objects and advantages of the present invention will become more apparent from the following detailed description, the appended claims, and the accompanying drawings.

### Technical Solution

To achieve the above objectives, in one aspect of this invention, there is provided an antibody or an antigen-binding fragment thereof that specifically binds to CD73, comprising a heavy chain variable region comprising an HCDR1 having the amino acid sequence selected from the group consisting of SEQ ID NOs: 1 to 9 and 78 to 80; an HCDR2 having the amino acid sequence selected from the group consisting of SEQ ID NOs: 10 to 18 and 81 to 83; and an HCDR3 having the amino acid sequence selected from the group consisting of SEQ ID NOs: 19 to 28 and 84 to 86.

As used herein, the term "antibody" refers to a peptide molecule that specifically recognizes and binds to a specific epitope of CD73, and includes antigen-binding fragments of the antibody (immunologically active fragments) as well as full-length antibody molecules. A full-length antibody has a structure comprising two full-length light chains and two full-length heavy chains, each light chain being linked to a heavy chain by a disulfide bond. The heavy chain constant region has the gamma (γ), mu (µ), alpha (α), delta (δ), and epsilon (ε) types and is subclassified into gamma1 (γ1), gamma2 (γ2), gamma3 (γ3), gamma4 (γ4), alpha1 (α1), and alpha2 (α2). The light chain constant region has kappa (κ) and lambda (λ) types.

As used herein, the term "antigen-binding fragment of an antibody" refers to a fragment of an antibody that retains substantial antigen-binding activity. Examples of antigen-binding fragments include, but are not limited to, Fab, F(ab'), F(ab')2, Fv, and nanobody.

Among antibody fragments, Fab is an antibody fragment having a single antigen-binding site and comprising the variable regions of the light chain and heavy chain, the constant region of the light chain, and the first constant region of the heavy chain (CH1).

Fab' differs from Fab in that it has a hinge region containing one or more cysteine residues at the C-terminus of the heavy chain CH1 domain. F(ab')2 antibodies are generated by formation of disulfide bonds between cysteine residues in the hinge regions of Fab'. Fv is the minimal antibody fragment consisting solely of the heavy chain variable region and the light chain variable region. Two-chain Fv consists of a heavy chain variable region and a light chain variable region linked by non-covalent interactions, while single-chain Fv generally comprises a heavy chain variable region and a light chain variable region linked covalently via a peptide linker, or directly at the C-terminus, thereby forming a dimer-like structure similar to two-chain Fv.

As used herein, the term "heavy chain" refers to both full-length heavy chains and fragments thereof comprising a variable region (VH) and three constant regions (CH1, CH2, and CH3), wherein the variable region comprises an amino acid sequence sufficient to confer specificity to an antigen.

As used herein, the term "complementarity determining region (CDR)" refers to the amino acid sequence of the hypervariable region of the heavy and light chains of an immunoglobulin. The heavy chain (HCDR1, HCDR2, and HCDR3) and light chain (LCDR1, LCDR2, and LCDR3) each contain three CDRs, which provide the major contact residues involved in binding to an antigen or epitope.

The scope of the antibody or antigen-binding fragment of the present invention includes variants having conservative amino acid substitutions in the CDR regions. Furthermore, the antibody or antigen-binding fragment of the present invention includes variants of the amino acid sequences disclosed in the sequence listing, provided that they are capable of specifically recognizing CD73. For example, additional changes can be made to the amino acid sequence of the antibody to further improve the binding affinity and/or other biological properties of the antibody. Such modifications include, for example, deletions, insertions, and/or substitutions of amino acid sequence residues of the antibody. These amino acid modifications are made based on the relative similarity of the amino acid side chain substituents, e.g., hydrophobicity, hydrophilicity, charge, size, etc. By analyzing the size, shape, and type of amino acid side chain substituents, it can be seen that arginine, lysine, and histidine are all positively charged residues; alanine, glycine, and serine have similar sizes; and phenylalanine, tryptophan, and tyrosine have similar shapes. Therefore, based on these considerations, arginine, lysine, and histidine; alanine, glycine, and serine; and phenylalanine, tryptophan, and tyrosine may be regarded as biologically equivalent.

In addition, amino acid substitutions in proteins that do not alter the activity of the molecule as a whole are known in the art (H. Neurath, R.L. Hill, The Proteins, Academic Press, New York, 1979). The most common exchanges are between the amino acid residues Ala/Ser, Val/Ile, Asp/Glu, Thr/Ser, Ala/Gly, Ala/Thr, Ser/Asn, Ala/Val, Ser/Gly, Thr/Phe, Ala/Pro, Lys/Arg, Asp/Asn, Leu/Ile, Leu/Val, Ala/Glu, and Asp/Gly.

Considering biologically equivalent variants as described above, the amino acid sequences of the antibody of the present invention may also encompass sequences having substantial identity to the sequences set forth in the sequence listing. Sequences having the substantial identity show at least 61% sequence identity, specifically at least 70%, more specifically at least 80% sequence identity, and most specifically at least 90% sequence identity to the sequences of present invention, when the sequences are aligned as closely as possible and analyzed using algorithms commonly employed in the art. Methods and algorithms for sequence alignment are disclosed in Huang et al., Comp. Appl. BioSci. 8:155-65(1992) and Pearson et al., Meth. Mol. Biol. 24:307-31(1994).

According to a specific embodiment, the antibody or the antigen-binding fragment thereof further comprises a light chain variable region comprising an LCDR1 having the amino acid sequence selected from the group consisting of SEQ ID NOs: 39 to 48; an LCDR2 having the amino acid sequence selected from the group consisting of SEQ ID NOs: 49 to 57; and LCDR3 having the amino acid sequence selected from the group consisting of SEQ ID NOs: 58 to 67.

As used herein, the term "light chain" refers to both full-length light chain and fragments thereof comprising a variable region VL and a constant region CL with the amino acid sequence having sufficient variable region sequence to confer specificity to an antigen.

According to a specific embodiment, the antibody or the antigen-binding fragment thereof is selected from the group consisting of the following (a) to (j):
(a) an antibody or antigen-binding fragment thereof comprising a heavy chain variable region comprising HCDR1 having the amino acid sequence of SEQ ID NO: 1, HCDR2 having the amino acid sequence of SEQ ID NO: 10, and HCDR3 having the amino acid sequence of SEQ ID NO: 19; and a light chain variable region comprising LCDR1 having the amino acid sequence of SEQ ID NO: 39, LCDR2 having the amino acid sequence of SEQ ID NO: 49, and LCDR3 having the amino acid sequence of SEQ ID NO: 58;
(b) an antibody or antigen-binding fragment thereof comprising a heavy chain variable region comprising HCDR1 having the amino acid sequence of SEQ ID NO: 2, HCDR2 having the amino acid sequence of SEQ ID NO: 11, and HCDR3 having the amino acid sequence of SEQ ID NO: 20; and a light chain variable region comprising LCDR1 having the amino acid sequence of SEQ ID NO: 40, LCDR2 having the amino acid sequence of SEQ ID NO: 50, and LCDR3 having the amino acid sequence of SEQ ID NO: 59;
(c) an antibody or antigen-binding fragment thereof comprising a heavy chain variable region comprising HCDR1 having the amino acid sequence of SEQ ID NO: 3, HCDR2 having the amino acid sequence of SEQ ID NO: 12, and HCDR3 having the amino acid sequence of SEQ ID NO: 21; and a light chain variable region comprising LCDR1 having the amino acid sequence of SEQ ID NO: 41, LCDR2 having the amino acid sequence of SEQ ID NO: 51, and LCDR3 having the amino acid sequence of SEQ ID NO: 60;
(d) an antibody or antigen-binding fragment thereof comprising a heavy chain variable region comprising HCDR1 having the amino acid sequence of SEQ ID NO: 4, HCDR2 having the amino acid sequence of SEQ ID NO: 13, and HCDR3 having the amino acid sequence of SEQ ID NO: 22; and a light chain variable region comprising LCDR1 having the amino acid sequence of SEQ ID NO: 42, LCDR2 having the amino acid sequence of SEQ ID NO: 52, and LCDR3 having the amino acid sequence of SEQ ID NO: 61;
(e) an antibody or antigen-binding fragment thereof comprising a heavy chain variable region comprising HCDR1 having the amino acid sequence of SEQ ID NO: 5, HCDR2 having the amino acid sequence of SEQ ID NO: 14, and HCDR3 having the amino acid sequence of SEQ ID NO: 23; and a light chain variable region comprising LCDR1 having the amino acid sequence of SEQ ID NO: 43, LCDR2 having the amino acid sequence of SEQ ID NO: 53, and LCDR3 having the amino acid sequence of SEQ ID NO: 62;
(f) an antibody or antigen-binding fragment thereof comprising a heavy chain variable region comprising HCDR1 having the amino acid sequence of SEQ ID NO: 6, HCDR2 having the amino acid sequence of SEQ ID NO: 15, and HCDR3 having the amino acid sequence of SEQ ID NO: 24; and a light chain variable region comprising LCDR1 having the amino acid sequence of SEQ ID NO: 44, LCDR2 having the amino acid sequence of SEQ ID NO: 54, and LCDR3 having the amino acid sequence of SEQ ID NO: 63;
(g) an antibody or antigen-binding fragment thereof comprising a heavy chain variable region comprising HCDR1 having the amino acid sequence of SEQ ID NO: 7, HCDR2 having the amino acid sequence of SEQ ID NO: 16, and HCDR3 having the amino acid sequence of SEQ ID NO: 25; and a light chain variable region comprising LCDR1 having the amino acid sequence of SEQ ID NO: 45, LCDR2 having the amino acid sequence of SEQ ID NO: 49, and LCDR3 having the amino acid sequence of SEQ ID NO: 64;
(h) an antibody or antigen-binding fragment thereof comprising a heavy chain variable region comprising HCDR1 having the amino acid sequence of SEQ ID NO: 8, HCDR2 having the amino acid sequence of SEQ ID NO: 17, and HCDR3 having the amino acid sequence of SEQ ID NO: 26; and a light chain variable region comprising LCDR1 having the amino acid sequence of SEQ ID NO: 46, LCDR2 having the amino acid sequence of SEQ ID NO: 55, and LCDR3 having the amino acid sequence of SEQ ID NO: 65;
(i) an antibody or antigen-binding fragment thereof comprising a heavy chain variable region comprising HCDR1 having the amino acid sequence of SEQ ID NO: 3, HCDR2 having the amino acid sequence of SEQ ID NO: 12, and HCDR3 having the amino acid sequence of SEQ ID NO: 27; and a light chain variable region comprising LCDR1 having the amino acid sequence of SEQ ID NO: 47, LCDR2 having the amino acid sequence of SEQ ID NO: 56, and LCDR3 having the amino acid sequence of SEQ ID NO: 66; and
(j) an antibody or antigen-binding fragment thereof comprising a heavy chain variable region comprising HCDR1 having the amino acid sequence of SEQ ID NO: 9, HCDR2 having the amino acid sequence of SEQ ID NO: 18, and HCDR3 having the amino acid sequence of SEQ ID NO: 28; and a light chain variable region comprising LCDR1 having the amino acid sequence of SEQ ID NO: 48, LCDR2 having the amino acid sequence of SEQ ID NO: 57, and LCDR3 having the amino acid sequence of SEQ ID NO: 67.

More specifically, the antibody or antigen-binding fragment thereof set forth in (a) comprises a heavy chain variable region having the amino acid sequence of SEQ ID NO: 29 and a light chain variable region having the amino acid sequence of SEQ ID NO: 68.

More specifically, the antibody or antigen-binding fragment thereof set forth in (b) comprises a heavy chain variable region having the amino acid sequence of SEQ ID NO: 30 and a light chain variable region having the amino acid sequence of SEQ ID NO: 69.

More specifically, the antibody or antigen-binding fragment thereof set forth in (c) comprises a heavy chain variable region having the amino acid sequence of SEQ ID NO: 31 and a light chain variable region having the amino acid sequence of SEQ ID NO: 70.

More specifically, the antibody or antigen-binding fragment thereof set forth in (d) comprises a heavy chain variable region having the amino acid sequence of SEQ ID NO: 32 and a light chain variable region having the amino acid sequence of SEQ ID NO: 71.

More specifically, the antibody or antigen-binding fragment thereof set forth in (e) comprises a heavy chain variable region having the amino acid sequence of SEQ ID NO: 33 and a light chain variable region having the amino acid sequence of SEQ ID NO: 72.

More specifically, the antibody or antigen-binding fragment thereof set forth in (f) comprises a heavy chain variable region having the amino acid sequence of SEQ ID NO: 34 and a light chain variable region having the amino acid sequence of SEQ ID NO: 73.

More specifically, the antibody or antigen-binding fragment thereof set forth in (g) comprises a heavy chain variable region having the amino acid sequence of SEQ ID NO: 35 and a light chain variable region having the amino acid sequence of SEQ ID NO: 74.

More specifically, the antibody or antigen-binding fragment thereof set forth in (h) comprises a heavy chain variable region having the amino acid sequence of SEQ ID NO: 36 and a light chain variable region having the amino acid sequence of SEQ ID NO: 75.

More specifically, the antibody or antigen-binding fragment thereof set forth in (i) comprises a heavy chain variable region having the amino acid sequence of SEQ ID NO: 37 and a light chain variable region having the amino acid sequence of SEQ ID NO: 76.

More specifically, the antibody or antigen-binding fragment thereof set forth in (j) comprises a heavy chain variable region having the amino acid sequence of SEQ ID NO: 38 and a light chain variable region having the amino acid sequence of SEQ ID NO: 77.

According to a specific embodiment, the antigen-binding fragment is a single-domain antibody.

As used herein, the term "single-domain antibody", also called a "nanobody", refers to a fragment of a full-length antibody comprising, in monomeric form, a single variable domain of an antibody. The single-domain antibody is able to selectively bind to a target antigen, as does a full-length antibody, despite having a molecular weight of about 12 to 15 kDa, which is smaller than that of an intact full-length antibody having a molecular weight of about 150 to 160 kDa.

More specifically, the single-domain antibody comprises an HCDR1 having the amino acid sequence selected from the group consisting of SEQ ID NOs: 78 to 80; an HCDR2 having the amino acid sequence selected from the group consisting of SEQ ID NOs: 81 to 83; and an HCDR3 having the amino acid sequence selected from the group consisting of SEQ ID NOs: 84 to 86.

According to a specific embodiment, the single-domain antibody is selected from the group consisting of the following (i) to (iii):
(i) a single-domain antibody comprising HCDR1 having the amino acid sequence of SEQ ID NO: 78, HCDR2 having the amino acid sequence of SEQ ID NO: 81, and HCDR3 having the amino acid sequence of SEQ ID NO: 84;
(ii) a single-domain antibody comprising HCDR1 having the amino acid sequence of SEQ ID NO: 79, HCDR2 having the amino acid sequence of SEQ ID NO: 82, and HCDR3 having the amino acid sequence of SEQ ID NO: 85; and
(iii) a single-domain antibody comprising HCDR1 having the amino acid sequence of SEQ ID NO: 80, HCDR2 having the amino acid sequence of SEQ ID NO: 83, and HCDR3 having the amino acid sequence of SEQ ID NO: 86.

More specifically, the single-domain antibody comprises the amino acid sequence selected from the group consisting of SEQ ID NOs: 87 to 89.

According to a specific embodiment, the single-domain antibody set forth in (i) comprises the amino acid sequence of SEQ ID NO: 87.

According to a specific embodiment, the single-domain antibody set forth in (ii) comprises the amino acid sequence of SEQ ID NO: 88.

According to a specific embodiment, the single-domain antibody set forth in (iii) comprises the amino acid sequence of SEQ ID NO: 89.

In another aspect of this invention, there is provided a nucleic acid molecule encoding the above-described antibody or the antigen-binding fragment thereof that specifically binds to CD73 protein.

As used herein, the term "nucleic acid molecule" is meant to encompass DNA (gDNA and cDNA) and RNA molecules. Nucleotides, which are the basic structural units in nucleic acid molecules, include not only natural nucleotides, but also analogues having modified sugar or base moieties (Uhlman and Peyman, Chemical Reviews, 90:543-584 (1990)). It will be clear to those skilled in the art that the nucleotide sequence encoding the amino acid sequence of an antibody or a heavy chain, light chain variable region thereof, may involve modifications including additions, deletions, or non-conservative or conservative substitutions of nucleotides.

The nucleic acid molecule of the present invention is interpreted to include a nucleotide sequence that is substantially identical to any of the above nucleotide sequences. Substantial identity means a necleotide sequence showing at least 80% sequence identity, specifically at least 90% sequence identity, more specifically at least 95% sequence identity as determined by aligning the sequence of the present invention with any other sequence to correspond to each other as much as possible and analyzing the aligned sequence using an algorithm commonly used in the art.

According to a specific embodiment, the nucleic acid molecule of the present invention may be an mRNA molecule, more specifically an in vitro transcribed (IVT) mRNA.

When mRNA is used as the nucleic acid molecule of the present invention, various modifications may be made to improve the efficiency of expression (translation) of the antibody or the antigen-binding fragment, including, for example, changes in the length of the poly(A) tail or substitution of some adenine bases; modification of the 5'cap; application of one or more modified nucleosides. The modified nucleosides include, for example, N1-methylpseudouridine, pseudouridine, 2-thiouridine, 5-methyluridine, , 5-methylcytidine, and 5-methoxyuridine, but are not limited thereto. Any modified nucleoside known in the art to reduce the immunogenicity of an mRNA molecule may also be used.

In still another aspect of this invention, there is provided a gene delivery system comprising the above-described nucleic acid molecule of the present invention.

According to the present invention, the antibody or the antigen-binding fragment thereof of the present invention may be recombinantly obtained *in vitro* by expressing a nucleic acid molecule encoding the antibody or antigen-binding fragment thereof in a host cell, or the nucleic acid molecule itself may be delivered in vivo into a patient as a pharmaceutically active ingredient to generate the antibody or the antigen-binding fragment thereof of the present invention in the body via the patient's expression system.

As used herein, the term "express" refers to being artificially replicated as an extrachromosomal factor or by chromosomal integration in a target cell via a gene delivery system to cause the target cell to express an exogenous gene or overexpress an endogenous gene. Accordingly, ter term "expression" may be used interchangeably with "transformation", "transfection", or "transduction". More specifically, in the present invention, the term "express" means causing a target cell to artificially express an exogenous gene.

The term "gene delivery system" or "gene delivery vehicle" as used herein refers to any means for delivering a gene into a cell, and gene delivery has the same meaning as intracellular transduction of the gene. At the tissue level, gene delivery has the same meaning as spread of the gene. Thus, the gene delivery system of the present invention may be referred to as a gene transduction system or a gene spread system.

The gene delivery system of the present invention may comprise, in the form of an expression cassette, a polynucleotide construct comprising all elements necessary for autonomous expression of a gene to be introduced. The expression cassette generally comprises a promoter, a transcription termination signal, a ribosome binding site, and a translation termination signal, which are operably linked to the gene. The expression cassette may be in the form of a self-replicable expression vector.As used herein, the term "operably linked" refers to a functional linkage between a nucleic acid expression regulatory sequence, such as a promoter, a signal sequence, or an array of transcription regulatory factor binding sites, and another nucleic acid sequence. Through the linkage, the expression regulatory sequence regulates the transcription and/or translation of the other nucleic acid sequence.

The recombinant vector system of the present invention can be constructed using various methods known to those skilled in the art, and specific methods for this are disclosed, for example, in Sambrook et al., in Molecular Cloning: A Laboratory Manual, Cold Spring Harbor Laboratory Press (2001).

The vectors of the present invention can typically be constructed as cloning vectors or expression vectors. Furthermore, the vectors of the present invention can be constructed using prokaryotic or eukaryotic cells as hosts. For example, if the vectors of the present invention are expression vectors using prokaryotic cells as host cells, a strong promoter capable of driving transcription, such as tac promoter, lac promoter, lacUV5 promoter, lpp promoter, pLλ promoter, pRλ promoter, rac5 promoter, amp promoter, recA promoter, SP6 promoter, trp promoter, and T7 promoter, and the like, a ribosome binding site for initiation of translation transcription/translation termination sequences are generally contained. When *E. coli* (e.g., HB101, BL21, DH5α, etc.) is used as the host cell, the promoter and operator sites of the *E. coli* tryptophan biosynthetic pathway (Yanofsky, C. J. Bacteriol. 158:1018-1024 (1984)) and the leftward promoter of phage λ (pLλ promoter, Herskowitz, I. and Hagen, D. Ann. Rev. Genet. 14:399-445 (1980)) can be utilized as regulatory sites. If Bacillus is utilized as the host cell, the promoter of the toxin protein gene of *Bacillus churrigensis* (Appl. Environ. Microbiol. 64:3932-3938 (1998); Mol. Gen. Genet. 250:734-741 (1996)) or any promoter that is expressible in *Bacillus* can be used as a regulatory site.

The vector expressing the antibody of the present invention may be either a vector system in which the light chain and heavy chain are simultaneously expressed from a single vector, or a system in which the light chain and heavy chain are respectively expressed from separate vectors. In the latter case, the two vectors may be introduced into a host cell through co-transformation or targeted transformation. Co-transformation refers to a method in which vector DNAs respectively encoding the light chain and the heavy chain are simultaneously introduced into a host cell, followed by selection of cells expressing both the light chain and the heavy chain. Targeted transformation refers to a method in which cells transformed with a vector encoding the light chain (or heavy chain) are first selected, and the selected cells expressing the light chain are subsequently re-transformed with a vector encoding the heavy chain (or light chain), thereby ultimately selecting cells expressing both the light chain and the heavy chain.

In still another aspect of this invention, there is provided a host cell transformed with the gene delivery system of the present invention.

Any host cell known in the art can be used as a host cell capable of stably and continuously cloning and expressing the gene delivery system of the present invention. For example, in the case of prokaryotic cells, examples thereof include, but are not limited to, *Escherichia coli*, the strains of the genus *Bacillus* such as *Bacillus subtilis* and *Bacillus thuringiensis*, *Streptomyces*, *Pseudomonas* (e.g., *Pseudomonas putida*), *Proteus mirabilis* and *Staphylococcus* (e.g., *Staphylococcus carnosus*).

The suitable eukaryotic host cells that may be used in the present invention include, for example, fungi such as *Aspergillus* species; yeasts such as *Pichia pastoris*, *Saccharomyces cerevisiae* and *Schizosaccharomyces;* insect-derived cells; plant-derived cells; or mammalian-derived cells such as CHO (Chinese hamster ovary) cells.

The culture of host cells for the production of antibodies or antigen-binding fragments thereof can be accomplished using any suitable medium and culture conditions known in the art. These culture procedures can be easily adapted by those skilled in the art to the strain selected, and examples of specific culture methods are disclosed in the literatures (e.g., James M. Lee, Biochemical Engineering, Prentice-Hall International Editions, 138-176). Antibodies obtained by host cell culture can be used in an un-purified state or can be further purified to high purity using a variety of conventional methods, e.g., dialysis, salt precipitation, and chromatography. When chromatography is used, the type and sequence of columns can be selected from ion-exchange chromatography, size-exclusion chromatography, affinity chromatography, etc. depending on the nature of the antibody and the method of host cell culture.

In still another aspect of this invention, there is provided an antibody or an antigen-binding fragment thereof that specifically binds to CD73 protein through one or more epitopes selected from the group consisting of (a) an epitope comprising the amino acid sequence of SEQ ID NO: 92; (b) an epitope comprising the amino acid sequence of SEQ ID NO: 93; and (c) an epitope comprising the amino acid sequence of SEQ ID NO: 94.

According to the present invention, the antibody or the antigen-binding fragment thereof of the present invention may serve as an excellent alternative treatment for patients exhibiting resistance or low responsiveness to Oleclumab, a commercially available anti-CD73 monoclonal antibody, by recognizing an epitope different from the epitopes of Oleclumab, i.e., YLPYKVLPVGDEV and KLKTLNVN.

In still another aspect of this invention, there is provided a pharmaceutical composition for preventing or treating cancer, comprising, as an active ingredient, the above-described antibody or the antigen-binding fragment thereof, nucleic acid molecule, or gene delivery system of the present invention.

As used herein, the term "preventing" refers to inhibiting the occurrence of a disorder or disease in a subject who has never been diagnosed as having the disorder or disease, but is at risk of developing the disorder or disease.

As used herein, the term "treating" refers to (a) inhibiting the progression of a disorder, disease or symptom; (b) alleviating the disorder, disease or symptom; or (c) eliminating the disorder, disease or symptom. The composition of the present invention, when administered to a subject, inhibits adenosine production, suppresses angiogenesis, and blocks immune evasion mechanisms in cancer cells that specifically overexpress CD73 on their surface, thereby inhibiting, eliminating, or alleviating the progression of symptoms caused by the cancer. Therefore, the composition of the present invention may itself be a therapeutic composition, or may be applied as a therapeutic adjuvant for the disease by being administered together with other active ingredients. Accordingly, the term "treatment" or "therapeutic agent" in the present specification includes the meaning of "therapeutic aid" or "therapeutic adjuvant".

As used herein, the term "administer" or "administration" refers to the direct administration of a therapeutically effective amount of the composition of the present invention to a subject so that an equal amount is formed in the body of the subject.

As used herein, the term "therapeutically effective amount" refers to an amount of the pharmaceutical composition of the present invention that is sufficient to provide a therapeutic or prophylactic effect to a subject to whom the composition is to be administered, and thus include the meaning of a "prophylactically effective amount".

As used herein, the term "subject" includes, without limitation, humans, mice, rats, guinea pigs, dogs, cats, horses, cows, pigs, monkeys, chimpanzees, baboons or rhesus monkeys. Specifically, the subject of the present invention is humans.

According to a specific embodiment, the cancer to be prevented or treated with the composition of the present invention is CD73-related cancer. "CD73-related cancer" refers to a malignant tumor that specifically overexpresses CD73 on the surface of cancer cells and whose symptoms can be improved or eliminated by inhibiting the expression or activity of CD73.

As used herein, the term "inhibition of expression or activity" refers to causing a reduction in the activity or expression of the CD73 protein, and refers not only to cases where the activity or expression of CD73 becomes undetectable or present at insignificant levels, but also to a reduction in the activity or expression of CD73 sufficient to significantly inhibit the conversion of AMP to adenosine and the immunosuppressive effects mediated by CD73.

More specifically, the cancer to be prevented or treated by the composition of the present invention is a cancer selected from the group consisting of leukemia, bladder cancer, brain tumor, melanoma, thyroid cancer, esophageal cancer, lung cancer, prostate cancer, breast cancer, colorectal cancer, gastric cancer, head and neck cancer, renal cancer, pancreatic cancer, liver cancer, neuroblastoma, retinoblastoma, salivary gland cancer, lymphoma, cervical cancer, ovarian cancer, and testicular cancer.

In still another aspect of this invention, there is provided a composition for detecting CD73-expressing cells, comprising the antibody or the antigen-binding fragment thereof of the present invention as an active ingredient.

According to the present invention, the antibodies of the present invention specifically recognize cells, particularly cancer cells, that specifically express CD73 on their surface, therefore may be used to selectively isolate, detect and quantify CD73-expressing cells in biological samples containing various cell populations. The term "detection" is therefore used interchangeably with "isolation", "sorting" and "quantification".

In still another aspect of this invention, there is provided a composition for diagnosing a cancer comprising the antibody or the antigen-binding fragment thereof of the present invention as an active ingredient.

As used herein, the term "diagnosis" includes a determination of susceptibility of a subject to a particular disease, a determination of whether a subject currently has a particular disease, and a determination of the prognosis of a subject with a particular disease.

As used herein, the term "a composition for diagnosing" refers to an integrated mixture or device comprising a means for measuring the expression level of CD73 protein to determine the presence of, or predict the likelihood of developing, a pathological condition predictable based on the expression level of CD73, such as a CD73-related cancer, and may also be referred to as a "diagnostic kit".

In still another aspect of this invention, there is provided a method for preventing or treating cancer, comprising administering the above-described antibody or the antigen-binding fragment of the present invention described above to a subject in need thereof.

In still another aspect of this invention, there is provided a composition for use in preventing or treating cancer, wherein the composition comprises the antibody or the antigen-binding fragment thereof of the present invention.

### Advantageous Effects

The features and advantages of the present invention are summarized as follows:
(a) The present invention provides an antibody or an antigen-binding fragment thereof that specifically binds to CD73 protein, and a nucleic acid molecule encoding the same.
(b) The antibody of the present invention binds with high affinity to CD73, a glycoprotein specifically overexpressed on the surface of tumor cells, thereby suppressing excessive angiogenesis in the tumor microenvironment, inhibiting adenosine production, reducing the activity of immunosuppressive cells such as regulatory T cells (Tregs), and enhancing the activity of immune cells such as T cells, thereby efficiently inducing the death of tumor cells.
(c) The antibody of the present invention exhibits CD73-binding ability and inhibitory activity equivalent or superior to those of Oleclumab, a commercially available anti-CD73 antibody. In particular, the anti-CD73 single-domain antibody of the present invention exhibits approximately 1.5- to 5-fold greater CD73 inhibitory activity compared to Oleclumab, thus may function as effective alternative therapeutics for patients with low responsiveness to Oleclumab.

### Brief Description of Drawings

FIG. 1 is a graph showing the inhibitory activity of the monoclonal antibodies CSA0055 to CSA0074 and Oleclumab against the enzymatic activity of soluble CD73.
FIG. 2 is a graph showing the inhibitory activity of the monoclonal antibodies CSA0075 to CSA0135 and Oleclumab against the enzymatic activity of soluble CD73.
FIG. 3 is a graph showing the inhibitory activity of the monoclonal antibodies CSA0160 to CSA0247 and Oleclumab against the enzymatic activity of soluble CD73.
FIG. 4 is a graph showing the inhibitory activity of the 10 produced single-domain antibodies and Oleclumab against the enzymatic activity of soluble CD73.
FIG. 5a is a diagram showing the epitope mapping results for the monoclonal antibody (CSA0060) and the single-domain antibodies (AHF10240, AHP04167).
FIG. 5b is a diagram showing the epitope of Oleclumab.
FIG 6 is a graph showing the proliferation of CD8+ T cells after CD8+ T cells whose activity had been inhibited by CD73-adenosine were treated with Vehicle (hIgG4), the monoclonal antibody (CSA0060), and Oleclumab.
FIG 7 is a graph showing the proliferation of CD4+ T cells after CD4+ T cells whose activity had been inhibited by CD73-adenosine were treated with Vehicle (hIgG4), the monoclonal antibody (CSA0060), and Oleclumab.

### Mode for Invention

Hereinafter, the present invention will be described in more detail by way of examples. These examples are only for illustrating the present invention in more detail, and it will be apparent to those skilled in the art that the scope of the present invention according to the subject matter of the present invention is not limited by these examples.

### Example 1. Screening of Anti-CD73 Monoclonal Antibodies (mAbs)

The anti-CD73 monoclonal antibodies (mAbs) were custom-made using an antibody screening service provided by Y-Biologics Co., Ltd., which screens antibodies from its human antibody library Ymax^{®}-ABL. The human antibody screening process utilized human CD73 antigens (CD73-His, CD73-Fc) and a human single-chain variable fragment (scFv) phage library to perform a total of 24 rounds of panning using immunotube panning, bead panning, and cell panning methods, thereby selecting 10,600 colonies. Monophage ELISA was performed on the 10,600 selected colonies to identify 1,475 hit clones, and sequence analysis of these 1,475 hit clones was conducted to identify 227 independent clones. Subsequently, phage FACS was performed to screen 61 monoclonal phage antibodies that bind to CD73-positive cells from the 227 independent clones.

Thereafter, following IgG conversion of the 61 monoclonal phage antibodies, an ELISA binding affinity test, Cell FACS, and a soluble CD73 blockade assay were performed.

### Example 2. Production of Anti-CD73 Monoclonal antibodies (mAbs)

To convert the monoclonal phage antibodies screened in Example 1 from the scFv form to the IgG, an additional request for the IgG conversion process was made to Y-Biologics Co., Ltd. For the IgG conversion process, the nucleotide sequences of the heavy chain variable regions of the 61 antibodies were cloned into pNATVH (Y-Biologics Co., Ltd.) using the restriction enzyme sites SfiI/NheI to generate the N293F HC vector. The light chain variable region sequences were cloned into the pNATVL vector (Y-Biologics Co., Ltd.) using the restriction enzyme sites SfiI/BglII to produce the N293F LC vector.

The cloned N293F HC vector and N293F LC vector were co-transfected into HEK293F cells, and the supernatant was collected on day 7. After centrifugation and filtration through a 0.22 µm Top-filter to remove cells and suspended particles, and the supernatant was collected and purified using protein A beads.

Subsequently, Protein A chromatography and SDS-PAGE were performed on the 61 monoclonal antibodies to analyze production-related parameters, such as concentration, purity and yield (Table 1).

**[Table 1]**

| No. | ID | Concentration (mg/mL) | Mass (mg) | Yield (mg/L) |
|---|---|---|---|---|
| 1 | CSA0055 | 0.06 | 0.16 | 3.89 |
| 2 | CSA0056 | 0.65 | 1.55 | 38.80 |
| 3 | CSA0057 | 0.42 | 0.85 | 21.22 |
| 4 | CSA0058 | 0.48 | 1.26 | 31.52 |
| 5 | CSA0059 | 1.48 | 3.54 | 88.55 |
| 6 | CSA0060 | 1.00 | 2.40 | 60.06 |
| 7 | CSA0061 | 0.54 | 1.23 | 30.77 |
| 8 | CSA0062 | 1.90 | 4.19 | 104.65 |
| 9 | CSA0063 | 1.05 | 3.66 | 91.59 |
| 10 | CSA0064 | 1.51 | 3.46 | 86.58 |
| 11 | CSA0065 | 1.66 | 5.32 | 132.90 |
| 12 | CSA0066 | 0.14 | 0.34 | 8.59 |
| 13 | CSA0067 | 0.56 | 1.29 | 32.35 |
| 14 | CSA0068 | 1.01 | 2.32 | 57.96 |
| 15 | CSA0069 | 1.71 | 5.98 | 149.46 |
| 16 | CSA0070 | 1.07 | 2.67 | 66.63 |
| 17 | CSA0071 | 0.62 | 1.54 | 38.59 |
| 18 | CSA0072 | 0.70 | 2.45 | 61.32 |
| 19 | CSA0073 | 0.71 | 2.28 | 56.92 |
| 20 | CSA0074 | 1.12 | 2.59 | 64.67 |
| 21 | CSA0075 | 0.79 | 2.76 | 69.07 |
| 22 | CSA0076 | 1.20 | 2.87 | 71.82 |
| 23 | CSA0077 | 1.87 | 6.55 | 163.64 |
| 24 | CSA0078 | 0.02 | 0.06 | 1.40 |
| 25 | CSA0079 | 1.29 | 3.88 | 96.98 |
| 26 | CSA0080 | 0.97 | 3.38 | 84.49 |
| 27 | CSA0081 | 0.28 | 0.57 | 14.15 |
| 28 | CSA0082 | 1.52 | 4.72 | 118.09 |
| 29 | CSA0083 | 1.35 | 3.23 | 80.71 |
| 30 | CSA0084 | 0.04 | 0.09 | 2.31 |
| 31 | CSA0085 | 1.78 | 6.05 | 151.31 |
| 32 | CSA0086 | 0.43 | 1.04 | 25.96 |
| 33 | CSA0127 | 0.08 | 0.20 | 4.91 |
| 34 | CSA0128 | 1.22 | 3.67 | 91.81 |
| 35 | CSA0129 | 2.36 | 5.91 | 147.67 |
| 36 | CSA0130 | 0.25 | 0.62 | 15.41 |
| 37 | CSA0131 | 2.32 | 1.16 | 28.96 |
| 38 | CSA0132 | 1.93 | 5.80 | 145.05 |
| 39 | CSA0133 | 1.74 | 5.23 | 130.85 |
| 40 | CSA0134 | 1.92 | 4.79 | 119.77 |
| 41 | CSA0135 | 1.63 | 4.07 | 101.68 |
| 42 | CSA0160 | 1.04 | 3.96 | 99.11 |
| 43 | CSA0161 | 0.30 | 1.16 | 28.93 |
| 44 | CSA0162 | 0.95 | 3.59 | 89.86 |
| 45 | CSA0163 | 0.43 | 1.30 | 32.54 |
| 46 | CSA0164 | 1.01 | 3.84 | 95.96 |
| 47 | CSA0165 | 1.27 | 4.81 | 120.30 |
| 48 | CSA0166 | 1.03 | 3.93 | 98.21 |
| 49 | CSA0167 | 1.29 | 4.89 | 122.29 |
| 50 | CSA0236 | 0.13 | 0.35 | 8.67 |
| 51 | CSA0237 | 0.48 | 1.29 | 32.25 |
| 52 | CSA0238 | 0.18 | 0.49 | 12.37 |
| 53 | CSA0239 | 0.11 | 0.31 | 7.75 |
| 54 | CSA0240 | 0.37 | 1.00 | 25.06 |
| 55 | CSA0241 | 0.17 | 0.46 | 11.57 |
| 56 | CSA0242 | 0.01 | 0.03 | 0.82 |
| 57 | CSA0243 | 0.27 | 0.72 | 17.93 |
| 58 | CSA0244 | 0.08 | 0.21 | 5.14 |
| 59 | CSA0245 | 0.01 | 0.03 | 0.87 |
| 60 | CSA0246 | 0.2 | 0.55 | 13.81 |
| 61 | CSA0247 | 0.28 | 0.76 | 19.11 |

### Example 3. Characterization of the Anti-CD73 Monoclonal Antibodies (mAbs)

### Example 3-1. ELISA Binding Affinity Test

100 ng of His-tagged CD73 was added to the wells of Pierce^{™} Nickel Coated Plates (#15142, Thermo Scientific^{™}) and coated at room temperature for 1 hour. Human CD73-His (Y-Biologics Co., Ltd.) was used as the antigen. After washing three times with washing solution (PBS containing 0.05% Tween-20 (#P9416, Sigma-Aldrich)), 300 µL of blocking solution (4% skim milk (#232120, BD Difco, USA)/PBS) was added to each well according to the protocol, and the plates were incubated at room temperature for 1 hour to block nonspecific binding.

61 monoclonal antibodies, serially diluted in a fixed dilution ratio from 100 nM to 0.5 pM, were added to each well and incubated at room temperature for 1 hour. After washing, HRP-labeled anti-human kappa antibody (#A7164, Sigma) was added and incubated at room temperature for 1 hour. At this time, Oleclumab, an anti-CD73 antibody, was used as the control. After washing five times with wash buffer, TMB substrate solution (#T0440-1L, Sigma) was added, and the reaction was allowed to proceed in the dark at room temperature for at least 3 minutes to confirm color development. The reaction was then stopped by adding sulfuric acid solution (#S1478, Samchun). Absorbance was measured at a wavelength of 450 nm using a spectrophotometer (#GM3000, Promega or SpectraMax M5, Molecular Devices). The binding affinities of the 61 monoclonal antibodies and the control antibody to CD73-His are shown in Tables 2, 3, and 4 below.

**[Table 2]**

| Antibody | Binding Affinity | |
|---|---|---|
| | KD | R-squared |
| IgG-Oleclumab | 1.2×10⁻¹¹ | 0.9912 |
| CSA0055 | 1.8×10⁻⁸ | 0.6369 |
| CSA0056 | 4.2×10⁻¹¹ | 0.9152 |
| CSA0057 | 3.6×10⁻⁹ | 0.9771 |
| CSA0058 | 3.3×10⁻⁹ | 0.9106 |
| CSA0059 | 7.8×10⁻⁹ | 0.9355 |
| CSA0060 | 5.6×10⁻¹¹ | 0.9943 |
| CSA0061 | 22×10⁻⁹ | 0.8108 |
| CSA0062 | 1.5×10⁻⁹ | 0.9779 |
| CSA0063 | 2.2×10⁻⁹ | 0.9810 |
| CSA0064 | 1.5x 10⁻⁹ | 0.9800 |
| CSA0065 | 1.0×10⁻⁹ | 0.9674 |
| CSA0066 | 4.1×10⁻¹⁰ | 0.9646 |
| CSA0067 | 6.4×10⁻⁹ | 0.7846 |
| CSA0068 | 2.1×10⁻⁹ | 0.8236 |
| CSA0069 | 7.4×10⁻¹⁰ | 0.9810 |
| CSA0070 | 1.5×10⁻⁹ | 0.9831 |
| CSA0071 | 2.0×10⁻⁹ | 0.9846 |
| CSA0072 | 3.6×10⁻¹⁰ | 0.9669 |
| CSA0073 | 4.2×10⁻¹⁰ | 0.9857 |
| CSA0074 | 3.2×10⁻⁸ | 0.7176 |
| CSA0075 | 1.5×10⁻⁹ | 0.9677 |
| CSA0076 | 1.6×10⁻⁹ | 0.9750 |
| CSA0077 | 1.2×10⁻⁹ | 0.9832 |
| CSA0078 | 3.0×10⁻⁹ | 0.9842 |
| CSA0079 | 1.7×10⁻¹⁰ | 0.9921 |
| CSA0080 | 3.0×10⁻⁹ | 0.9501 |
| CSA0081 | 5.9×10⁻¹¹ | 0.9981 |
| CSA0082 | 2.5×10⁻⁹ | 0.7756 |
| CSA0083 | 4.2×10⁻⁹ | 0.6213 |
| CSA0084 | 6.2×10⁻⁹ | 0.9531 |
| CSA0085 | 1.2×10⁻⁹ | 0.9755 |
| CSA0086 | 2.5×10⁻⁹ | 0.9688 |
| CSA0127 | - | 0.09492 |
| CSA0128 | 1.0×10⁻⁸ | 0.8201 |
| CSA0129 | 1.0×10⁻⁸ | 0.5881 |
| CSA0130 | 3.9×10⁻¹¹ | 0.9979 |
| CSA0131 | 1.0×10⁻⁸ | 0.09028 |
| CSA0132 | 2.0×10⁻¹¹ | 0.9903 |
| CSA0133 | 1.9×10⁻⁹ | 0.9352 |
| CSA0134 | 1.0×10⁻⁸ | 0.1965 |
| CSA0135 | 7.5×10⁻¹⁰ | 0.9272 |

**[Table 3]**

| Antibody | Binding Affinity | |
|---|---|---|
| | KD | R-squared |
| IgG-Oleclumab | 1.0×10⁻¹¹ | 0.9926 |
| CSA0160 | 9.6×10⁻¹¹ | 0.9980 |
| CSA0161 | 6.4×10⁻¹⁰ | 0.9909 |
| CSA0162 | 3.1×10⁻⁹ | 0.9894 |
| CSA0163 | 5.6×10⁻⁹ | 0.8355 |
| CSA0164 | 9.5×10⁻¹⁰ | 0.9932 |
| CSA0165 | 3*.*4×10⁻⁹ | 0.9758 |
| CSA0166 | 9.5×10⁻¹⁰ | 0.9753 |
| CSA0167 | 5.1×10⁻¹¹ | 0.9933 |

**[Table 4]**

| Antibody | Binding Affinity | |
|---|---|---|
| | KD | R-squared |
| IgG-Oleclumab | 4.4×10⁻¹² | 0.9970 |
| CSA0236 | 4.1×10⁻⁹ | 0.9795 |
| CSA0237 | 2.9×10⁻⁹ | 0.9910 |
| CSA0238 | 3.6×10⁻¹⁰ | 0.9746 |
| CSA0239 | 4.3×10⁻¹⁰ | 0.9910 |
| CSA0240 | 1.1×10⁻⁹ | 0.9928 |
| CSA0241 | 1.6×10⁻⁹ | 0.9961 |
| CSA0242 | - | - |
| CSA0243 | 9.4×10⁻¹⁰ | 0.9615 |
| CSA0244 | - | - |
| CSA0245 | - | - |
| CSA0246 | 2.8×10⁻⁹ | 0.7182 |
| CSA0247 | 1.9×10⁻¹¹ | 0.9929 |

### Example 3-2. Cell FACS

FACS analysis was performed to confirm whether monoclonal antibodies (CSA0055 to CSA0086, CSA0127 to CSA0136, and CSA0236 to CSA0247) bind to CD73-positive cells (MDA-MB-231) and CD73-negative cells (Jurkat). CD73-positive cells (MDA-MB-231) and CD73-negative cells (Jurkat) were prepared at 0.5 x 10⁶ cells per sample, and the prepared cells were incubated with each of the 53 monoclonal antibodies at a concentration of 2 µg/mL for 30 minutes at 4°C. Subsequently, the cells were washed three times with PBS (#LB001-02, Welgene) containing 2% fetal bovine serum (#26140-079, Thermo), and then incubated with an anti-human IgG antibody conjugated with FITC (fluorescein isothiocyanate) (#F1-3000, Vectorlabs) or PE-anti-hIgG antibody (#555787, BD) at 4°C for 20 minutes, followed by washing as described above. The cells were resuspended in 0.5 mL of PBS containing 2% FBS and analyzed using a FACSCanto^{™} II flow cytometer (BD Biosciences, USA).

As a result, it was confirmed that 32 monoclonal antibodies (CSA0055 to CSA0086) bound to CD73-positive cells (MDA-MB-231), and that the monoclonal anti-CD73 antibodies CSA0071, CSA0073, CSA0075, and CSA0079 also bound to CD73-negative cells (Jurkat).

Furthermore, among the 9 monoclonal antibodies (CSA0127 to CSA0135), it was confirmed that the remaining 8 antibodies, excluding CSA0127, bound to CD73-positive cells. Furthermore, it was confirmed that the control antibody (Oleclumab) and the CSA0133 monoclonal antibody bound nonspecifically to CD73-negative cells.

Among the 12 monoclonal antibodies (CSA0236 to CSA0247), it was confirmed that the remaining 10 antibodies, excluding the CSA0244 and CSA0245, bound to CD73-positive cells.

### Example 3-3. Soluble CD73 blockade assay

The ability of the 61 monoclonal antibodies to inhibit soluble CD73 enzymatic activity was evaluated using the AMP-Glo^{™} Assay kit (Promega, V5012) according to the manufacturer's manual. Specifically, the 61 monoclonal antibodies and the control antibody Oleclumab were diluted to 0.05 ng/mL, dispensed at 2.5 µL per well into a 96-well plate, and 12.5 µL of 10 µM AMP was added to each well. Next, 10 µL of recombinant human CD73 protein (2.5 ng/mL) was added to each well, and the plates were incubated at room temperature for 10 minutes.

25 µl of AMP-Glo^{™} Reagent (Promega) was added to each well to stop the reaction; the plate was shaken for 2 minutes and then incubated at room temperature for 1 hour. AMP Detection solution was added to each well; the plate was shaken for 2 minutes and then incubated at room temperature for 1 hour. Luminescence was measured using the GloMax^{®}-Multi Detection System (Promega).

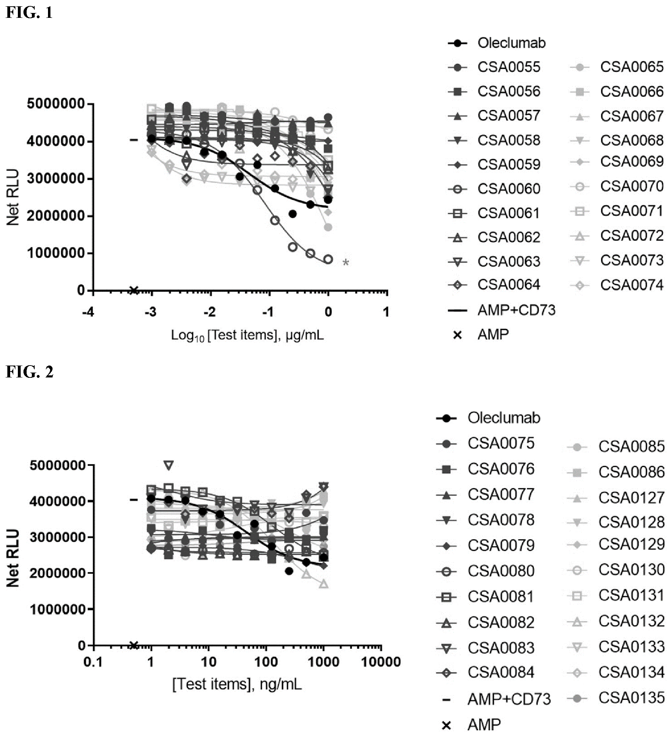

As a result, among the 61 monoclonal antibodies, 10 clones that effectively inhibited soluble CD73 enzymatic activity (CSA0060, CSA0065, CSA0073, CSA0074, CSA0081, CSA0132, CSA0135, CSA0238, CSA0240, CSA0247) were selected (FIGS. 1-3).

The SEQ ID NOs and specific amino acid sequences of the 10 selected clones are shown in Tables 5 and 6, respectively.

**[Table 5]**

| ID | SEQ ID NO | | | | | | | |
|---|---|---|---|---|---|---|---|---|
| | HCDR1 | HCDR2 | HCDR3 | Total VH | LCDR1 | LCDR2 | LCDR3 | All VL |
| CSA0060 | 1 | 10 | 19 | 29 | 39 | 49 | 58 | 68 |
| CSA0065 | 2 | 11 | 20 | 30 | 40 | 50 | 59 | 69 |
| CSA0073 | 3 | 12 | 21 | 31 | 41 | 51 | 60 | 70 |
| CSA0074 | 4 | 13 | 22 | 32 | 42 | 52 | 61 | 71 |
| CSA0081 | 5 | 14 | 23 | 33 | 43 | 53 | 62 | 72 |
| CSA0132 | 6 | 15 | 24 | 34 | 44 | 54 | 63 | 73 |
| CSA0135 | 7 | 16 | 25 | 35 | 45 | 49 | 64 | 74 |
| CSA0238 | 8 | 17 | 26 | 36 | 46 | 55 | 65 | 75 |
| CSA0240 | 3 | 12 | 27 | 37 | 47 | 56 | 66 | 76 |
| CSA0247 | 9 | 18 | 28 | 38 | 48 | 57 | 67 | 77 |
| constant region | Heavy-chain constant region: 90, Light-chain constant region: 91 | | | | | | | |

**[Table 6]**

| ID | Chain | Amino Acid Sequence | | | |
|---|---|---|---|---|---|
| | | Variable Region | CDR1 | CDR2 | CDR3 |
| CSA 0060 | **VH** | QVQLVQSGAEVKKPGSSVKVSCKFSGGS FTSYSFSWVRQAPGQGLEWMGRIIPVLT TTDYAQKFRDRVTITADESTSTAYMELS GLRSEDTAVYYCASGLKKNWYFDLWGR GALITVSS (29) | GGSFTSY S (1) | IIPVLTT T **(10)** | SGLKK NWYFD L (19) |
| | **VL** | DIQMTQSPSFLSASVGDRVTITCQASQDI SHYLNWYQQKPGKAPKLLIYDASSLETG VPSRFSGSGSGTSFTLTISSLQPEDFATYY CQQYDDFPLTFGGGTKVDIK (68) | QDISHY **(39)** | DAS **(49)** | QYDDFP LT (58) |
| CSA 0065 | **VH** | QVQLVQSGAEVKRPGESLRISCKTSGYSF TSYWIHWVRQMPGKELEWMGSIYPGNS DTRYSPSFQGQVTISADKSTSTAFLQWSS LEASDTAMYYCGRQDYYGSLKYWGQG TPITVSS (30) | GYSFTSY W (2) | IYPGNS DT (11) | RQDYY GSLKY (20) |
| | **VL** | QLVLTQPRSVSGSPGQSVTISCTGTNTDI GGYNFVSWYQQHPGKAPKLMIYDVTRR PSGVPDRFSGSKSDNTASLTISGLQPEDE ADYYCASYAGSHYLFGTGTKVTVL (69) | NTDIGGY NF (40) | DVT **(50)** | SYAGSH YL (59) |
| CSA 0073 | **VH** | QMQLVQSGAEVKKPGASVKLSCKASGY TFSSYWMHWVRQAPGQRLEWMGEINPG NGHTNYNEKFKSRVTITVDKSASTAYME LSSLRSEDTAVYYCVRDRVAVAGYGGV | GYTFSSY W (3) | INPGNG HT (12) | RDRVA VAGYG GVQLD S (21) |
| | | QLDSWGQGTLITVSS (31) | | | |
| | **VL** | DIQMTQSPSFLSASVGDRVTITCRASQYI GTYLAWYQQQPGKAPKLLIYGASTLQSG VPSRFSGSGSGTDFTLTISSLQPEDFASYY CQQGYSTPWTFGQGTKVEIK (70) | QYIGTY (41) | GAS **(51)** | QGYSTP WT (60) |
| CSA 0074 | **VH** | QVQLVESGGGRAQPGSSLRLSCAASGFS FDDYAMHWVRQVPGKGLEWVAGISWD SHHMNYADSLKGRITISRDNARNSVYLA MNSLRAEDTAVYYCVRVGRYYDPFDLW GRGTLVTVSS (32) | GFSFDDY A (4) | ISWDSH HM (13) | RVGRY YDPFDL 22) |
| | **VL** | DIQMTQSPSAMSASVGDRVTITCRASQDI NNYLAWFHQKPGKAPKRLIFSASILQSG VPSRFRGRGSGTQFTLTISSLQAEDSGTY FCLQHNTYPPTFGGGTKVEIK (71) | QDINNY (42) | SAS **(52)** | QHNTY PPT (61) |
| CSA 0081 | **VH** | QVQLVESGGGVVQPGRSLRLSCAASGFT FSNYGMHWVRQAPGKGLEWVASIWYD GSKNYYADSVKGRFTISRDNSKNTLYLE MNSLRAEDTAVYYCARDTRSGYYFEGL DIWGQGTRVTVSS (33) | GFTFSNY G (5) | IWYDGS KN (14) F I | RDTRSG YYFEGL DI (23) |
| | **VL** | DIQMTQSPSSLSASEGDTVTITCRASQNIY TYLNWYQQKPGKAPELLIYVASSLQSGV PSRFSGSGSGTDFTLTINSLQPEDSATYLC QQSHSQPLTFGGGTKVDIK (72) | QNIYTY (43) | VAS (53)I | QSHSQP LT (62) |
| CSA 0132 | **VH** | QVQLVESGGGVVQPGRSPRLSCAASGFT FRSYAMHWVRQAPGKGLEWVAVISYDG NNKYYANSVKGRFTISRDNSKNTLYLQ MNSLRAEDTAVYYCVRDRTDYFDLWG QGTLVTVSS (34) | GFTFRSY A (6) | ISYDGN NK (15) | RDRTD YFDL 24) |
| | **VL** | DIQMTQSPSSVSASVGDRVTITCRASQGI GSWLAWYQQKPGKAPKLLIYAASSLQN GVPSRFSGSGSGTDFTLTINSLQPEDFAT YYCQQVRSFPSTFGQGTKLDIK (73) | QGIGSW (44) | AAS **(54)** | QVRSFP ST (63) |
| CSA 0135 | **VH** | QVQLQESGPGLVKPSQTLSLTCSVSGASI SSGYYYWSWIRQPAGKGPEWIGRISTTG TDYNPSLKSRVIISLDTSKNQLSLTLNSVT AVDTAVYYCAREIGGAFDIWGQGTMITV SS (35) | GASISSG YYY (7) | ISTTGT **(16)** | REIGGA FDI (25) |
| | **VL** | DIQMTQSPSSLSASVGDRVTITCRASQSIS SYLNWYQQKPGKAPKLLIYDASNLETGV PSRFSGSGAGTSFTLTISSLQPEDFATYYC QQTYSIPLTFGGGTKVEIK (74) | QSISSY **(45)** | DAS **(49)** | QTYSIP LT (64) |
| CSA 0238 | **VH** | QVQLVESGGGVVQPGGSLRLSCAASGFT FSTYAMHWVRQAPGKGLEWVAVISYDG SNKYYADSVKGRFTISRDNSKNTLYLQM NSLRAEDTAVYYCARGYYDSSGYYWSD AFDIWGQGTMVTVSS (36) | GFTFSTY A (8) | ISYDGS NK (17) | RGYYD SSGYY WSDAF DI (26) |
| | **VL** | DIVMTQTPLSLPVTPGEPASISCKSSQSLL NSDDGNTYLDWYLQKPGQSPQLLIYTLS HRASGVPDRFSGSGSGTDFTLKISRVAAE DVGLYYCLQRMGFPYTFGQGTKVDIK (75) | QSLLNSD DGNTY (46) | TLS **(55)** | QRMGF PYT (65) |
| CSA 0240 | **VH** | QMQLVQSGAEVKKPGASVKLSCKASGY TFSSYWMHWVRQAPGQRLEWMGEINPG NGHTNYNEKFKSRVTITVDKSASTAYME LSSLGSEDTAVYYCAREDCSGGSCYYYG MDVWGQGTTVTVSS (37) | GYTFSSY W (3) | INPGNG HT (12) | REDCSG GSCYY YGMDV (27) |
| | **VL** | SYELTQPPSVSLSPGQTARITCSGDALSK EYSYWYQQKPGQAPVLVMYKDNVRPS GIPERFSGSSSGTTVTLTISRVQAEDEADY YCQSVDSSDTSVVFGGGTKLTVL (76) | ALSKEY **(47)** | KDN **(56)** | SVDSSD TSVV (66) |
| CSA 0247 | **VH** | QMQLVQSGAELKMPGSSVKVSCKASGY TFTSYGISWVRQAPGQGLEWMGWISAY NGNTNYAQKLQGRVTMTTDTSTSTAYM ELRSLRSEDTAVYYCARGVWGSGLYYF DYWGQGSLVTVSS (38) | GYTFTSY G (9) | ISAYNG NT (18) | RGVWG SGLYYF DY (28) |
| | **VL** | DIQMTQSPGTLSLSPGERATLSCGASRSV LNYLAWYQQKPGQPPRLLIYDATKRASD IPARFSGSRSGRDFTLTISSLEPEDSAVYY CQHYGSSSGYLFGQGTKVEIK (77) | RSVLNY **(48)** | DAT **(57)** | HYGSSS GYL (67) |
| consta nt region | **CH** (CH1-IgG1 | ASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDK KVEPKSCDKTHTCPPCPAPELLGGPSVFLFPPKPKDTLMISRTPEVTCV | | | |
| (Com mon) | Fc) | VVDVSHEDPEVKFNWYVDGVEVHNAKTKPREEQYNSTYRVVSVLTV LHQDWLNGKEYKCKVSNKALPAPIEKTISKAKGQPREPQVYTLPPSRE EMTKNQVSLTCLVKGFYPSDIAVEWESNGQPENNYKTTPPVLDSDGSF FLYSKLTVDKSRWQQGNVFSCSVMHEALHNHYTQKSLSLSPGK (90) | | | |
| | **CL** (kappa ) |  (91) | | | |

### Example 4. Screening of anti-CD73 single-domain antibodies (sdAbs)

The anti-CD73 single-domain antibodies were custom-made using GenScript ProBio's Single Domain Antibody Discovery Services. The single-domain antibody screening process followed GenScript ProBio's protocol, which involved immunizing a non-immunized alpaca by administering human CD73 antigen at approximately two-week intervals for four to six rounds. Blood samples were collected during each immunization round, and the immune response was monitored by ELISA and FACS. To construct the single-domain antibody library, total RNA was extracted from lymphocytes obtained from the immunized alpaca, and PCR was performed using primers (forward and reverse) to amplify VHH fragments. The PCR products were cloned into a Llama library vector to generate the single-domain antibody library.

Solid-phase panning was performed using the constructed single-domain antibody library to select colonies. Monophage ELISA and phage FACS were performed on the selected colonies to screen for 10 purified CD73 single-domain antibodies having high binding affinity to human CD73.

### Example 5. Production of anti-CD73 single-domain antibodies (sdAbs)

The nucleotide sequences of the single-domain antibodies screened in Example 4 were cloned into the pTT5 vector (GenScript) and co-transfected into Expi293F^{™} cells. Then, the culture supernatant was harvested and purified using protein A beads.

Subsequently, Protein A chromatography and SDS-PAGE were performed on the 10 single-domain antibodies to analyze production-related parameters such as concentration, purity, and yield (Table 7).

**[Table 7]**

| sdAb | Conc. (mg/mL) | Vol. (mL) | Mass (mg) | Purity (%) |
|---|---|---|---|---|
| AHF10235 | 3.723 | 4.2 | 15.637 | 99% |
| AHF10239 | 1.402 | 4.2 | 5.888 | 99% |
| AHF10240 | 3.183 | 3.8 | 12.095 | 99% |
| AHF10246 | 3.451 | 4.0 | 13.804 | 99% |
| AHF10294 | 1.055 | 4.2 | 4.431 | 99% |
| AHF10295 | 2.263 | 4.0 | 9.052 | 99% |
| AHP04048 | 1.777 | 4.0 | 7.108 | 95% |
| AHP04146 | 2.607 | 4.2 | 10.949 | 99% |
| AHP04148 | 2.999 | 4.2 | 12.596 | 99% |
| AHP04167 | 3.092 | 4.0 | 12.368 | 99% |

### Example 6. Characterization of anti-CD73 single-domain antibodies (sdAbs)

### Example 6-1. ELISA: EC₅₀

1 µg/mL of human CD73 (ACRO Biosystems) and 1 µg/mL of mouse CD73 (ACRO Biosystems) were each added at 100 µL per well to a 96-well plate and coated overnight at 4°C. After washing, a blocking solution of 3% skim milk in PBS was added, and the plate was incubated at room temperature for 1 hour to block nonspecific binding.

100 µL of each of 10 single-domain antibodies serially diluted at a fixed dilution ratio were added to each well and incubated at room temperature for 1 hour and 30 minutes. The wells were washed, HRP-labeled MonoRab^{™} Rabbit anti-camelid VHH Cocktail (A02016-200, GenScript) was added and incubated at room temperature for 45 minutes. After washing, TMB substrate solution was added, and the mixture was incubated in the dark at room temperature for at least 3 minutes to observe color development. Absorbance was measured at 450 nm using a spectrophotometer. The EC₅₀ values for the binding of the 10 single-domain antibodies to human CD73 and mouse CD73 were calculated and presented in Table 8 below.

**[Table 8]**

| **sdAb** | hCD73 EC₅₀ (µg/mL) | mCD73 EC₅₀ (µg/mL) |
|---|---|---|
| AHF10235 | 0.001644 | 0.006139 |
| AHF10239 | 0.002046 | 0.002623 |
| AHF10240 | 0.002377 | 0.006455 |
| AHF10246 | 0.001266 | 0.001944 |
| AHF10294 | 0.002045 | 0.002094 |
| AHF10295 | 0.002801 | 0.01015 |
| AHP04048 | 0.5286 | 3.075 |
| AHP04146 | 0.001966 | 0.002108 |
| AHP04148 | 0.001642 | 0.002070 |
| AHP04167 | 0.001721 | 0.004032 |
| NC | NA | NA |
| Blank | NA | NA |

### Example 6-2. Soluble CD73 blockade assay

The ability of the 10 single-domain antibodies to inhibit soluble CD73 enzymatic activity was evaluated using the AMP-Glo^{™} Assay kit (Promega, V5012) according to the manufacturer's manual. Specifically, the 10 single-domain antibodies and the control, Oleclumab, were diluted to 0.05 ng/mL and dispensed at 2.5 µL per well into a 96-well plate, and 12.5 µL of 10 µM AMP was added to each well. Subsequently, 10 µL of recombinant human CD73 protein (2.5 ng/mL) was added to each well, and the plates were incubated at room temperature for 10 minutes.

To terminate the reaction, 25 µl of AMP-Glo^{™} Reagent (Promega) was added to each well, and the plate was shaken for 2 minutes before being incubated at room temperature for 1 hour. AMP Detection Solution was added to each well, and the plate was shaken for 2 minutes, and then incubated at room temperature for 1 hour. Luminescence was measured using the GloMax^{®}-Multi Detection System (Promega), and the IC₅₀ values were calculated using GraphPad Prism 8.0 software (Table 9).

**[Table 9]**

| Category | IC₅₀ (ng/mL) |
|---|---|
| Oleclumab | 45.7 |
| AHF10235 | 16.9 |
| AHF10239 | 20.4 |
| AHF10240 | 5.5 |
| AHF10246 | Ambiguous (~ 3.661e+0.14) |
| AHF10294 | 29.9 |
| AHF10295 | 6.4 |
| AHP04048 | 703 |
| AHP04146 | 889 |
| AHP04148 | 1242 |
| AHP04167 | 7.5 |

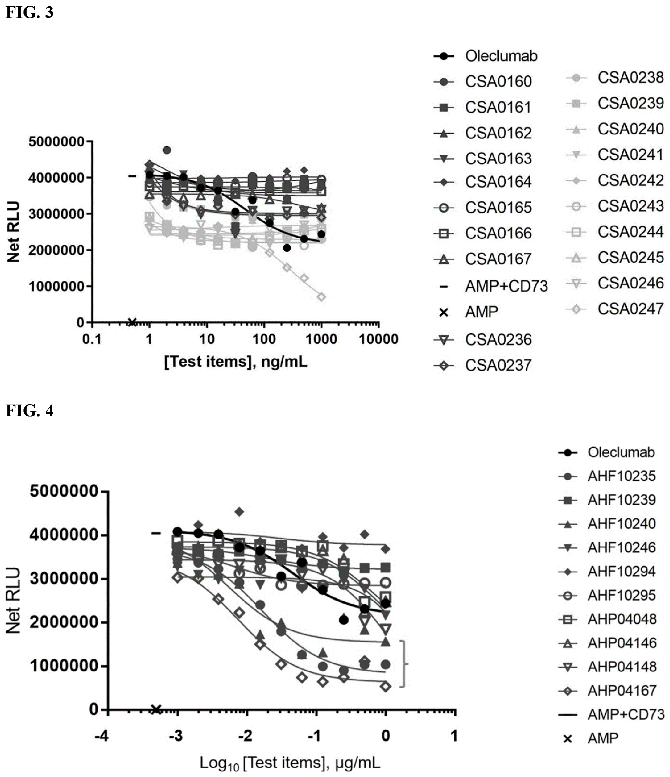

As a result, it was confirmed that three single-domain antibodies (AHF10235, AHF10240 and AHP0416) significantly inhibited soluble CD73 enzyme activity compared to the control, Oleclumab (FIG. 4). The IC₅₀ values of these three single-domain antibodies were determined to be 16.9 ng/mL (0.2 nM), 5.5 ng/mL (0.06 nM) and 7.5 ng/mL (0.08 nM), respectively, while the IC₅₀ value of Oleclumab was determined to be 45.7 ng/mL (0.3 nM).

Based on a comprehensive analysis of the results from Examples 5, 6-1 and 6-2, three single-domain antibodies (AHF10235, AHF10240 and AHP04167) were selected from the 10 single-domain antibodies. The SEQ ID NOs and specific amino acid sequences of the selected single-domain antibodies are shown in Table 10.

**[Table 10]**

| ID | VH | CDR1 | CDR2 | CDR3 |
|---|---|---|---|---|
| AHP | EVQLVESGGGLVQAGGSLRLSCVVSG | GRTFSNY | IGRSGGPT | AGDWRNSD |
| 04167 | RTFSNYHMGWFRQAPGKEREFVAVIG RSGGPTYYADSVKGRFTISRDNAKNT VYLQMNSLKPEDTAVYYCAGDWRNS DSPSKLKPVYDYWGQGTQVTVSS (87) | H **(78**) | **(81)** | SPSKLKPVY DY (84) |
| AHF10 235 | QVQVQESGGGLVQAGDSLRLSCAASG RSISRYNMGWFRQAPGKEREFVAAISR SGGVTYYADSVKGRLTISRDNAKSAV YLQMNSLKPEDTAVYYCAADWRNSD SPSPLIIVYDYWGQGTQVTVSS (88) | GRSISRYN **(79)** | ISRSGGVT **(82)** | AADWRNSD SPSPLIIVYD Y (85) |
| AHF10 240 | EVQLVESGGGVVQTGGSLRLSCAASG RTFTHLAMGWFRQAPGKEREFVAAIS NSGAGTQFADSVKGRFTISRDNAKNT VYLQMNSLKPEDTAVYYCAADWGTR DSPSKLNAVYDYWGRGTQVTVSS (89) | GRTFTHL A (80) | ISNSGAGT **(83)** | AADWGTRD SPSKLNAV YDY (86) |

### Example 7. Identification of the binding site of anti-CD73 antibodies (mAbs and sdAbs) on CD73

To determine the binding sites on CD73 for the monoclonal antibody (CSA0060) and single-domain antibodies (AHF10240, AHP04167) finally selected in Examples 3-3 and 6-2 above, AbSolute^{™} epitope mapping analysis was performed by Jinon Biotech using a peptide microarray.

Using the amino acid sequence of CD73, an antigen-specific peptide array comprising 160,000 spots-consisting of linear overlapping fragments (1,120 spots) and discontinuous structural fragments (158,880 spots)-was generated. Spots containing peptides with high affinity for the test antibody were scanned, visualized, and analyzed using a GenePix 4100A Microarray Scanner (Molecular Devices).

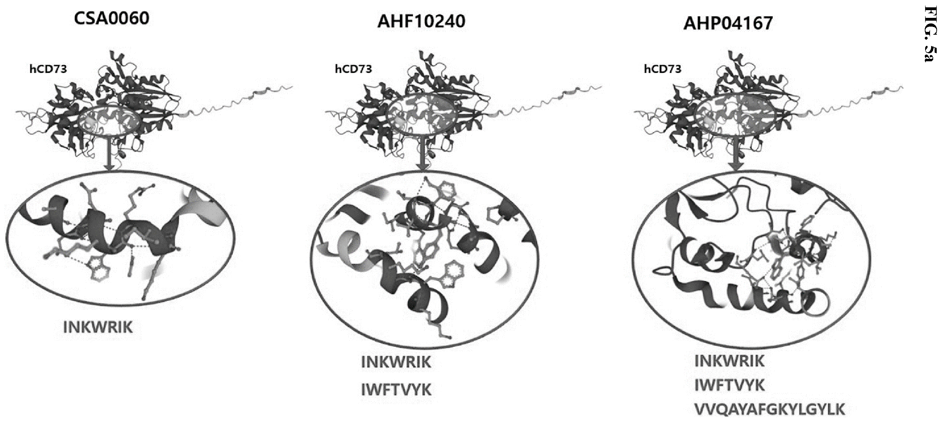

As a result, it was confirmed that a monoclonal antibody (CSA0060) and single-domain antibodies (AHF10240, AHP04167) recognize the highly flexible α-helical linker (INKWRIK) between the N- and C-terminal domains of CD73. Furthermore, it was confirmed that the single-domain antibodies (AHF10240, AHP04167) additionally recognize one epitope (IWFTVYK) and two epitopes (IWFTVYK and VVQAYAFGKYLGYLK), respectively (FIG. 5a).

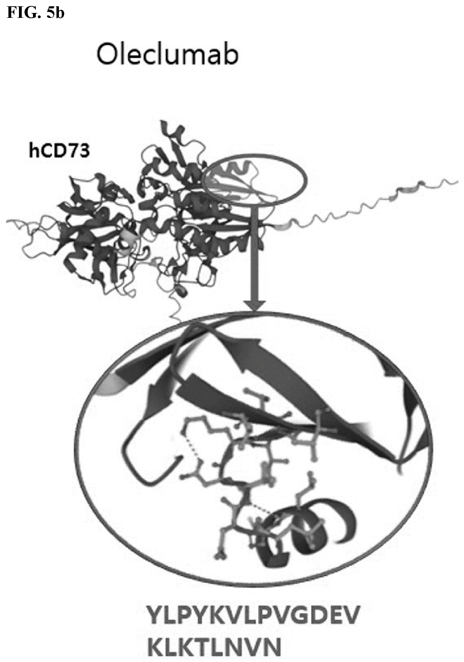

The control antibody Oleclumab was found to recognize two epitopes (YLPYKVLPVGDEV and KLKTLNVN) (James C. Geoghegan et al (2016), mAbs, 8:3, 454-467) (FIG. 5b). Therefore, Oleclumab has binding sites different from those of the monoclonal antibody and single-domain antibodies of the present invention.

### Example 8. Confirmation of reinvigoration of T cells inhibited by CD73-adenosine by the anti-CD73 monoclonal antibody (CSA0060)

To confirm whether the anti-CD73 monoclonal antibody (CSA0060) restores the activity of CD8+ and CD4+ T cells inhibited by CD73-adenosine, human PBMCs were cultured overnight in TexMACS^{™} medium (Miltenyi Biotec) containing 5% human serum (Sigma-Aldrich^{®}), 1% penicillin/streptomycin, and 5 mM β-mercaptoethanol (Thermo Fisher Scientific) at 37°C under humidified conditions with 5% CO₂. The following day, the cells were labeled with CTV and seeded into a 96-well plate at a density of 1 x 10⁶ cells/ml. The cells were stimulated using αCD3/CD28 Dynabeads (1:1 ratio).

The cells were treated with vehicle (hIgG4), anti-CD73 monoclonal antibody (CSA0060), and Oleclumab, respectively. Each agent was added to each well such that the final concentration was 50 nM. Subsequently, 500 µM AMP was added to each well, and the cells were cultured for 3 days at 37°C in humidified conditions with 5% CO₂.

To assess T cell functional activation, the cells were restimulated with αCD3/CD28 dynabeads (1:1 ratio), 50 nM or 500 µM AMP in the presence of Brefeldin A for 4 hours at 37°C under humidified conditions with 5% CO₂. To analyze Teff cell proliferation by FACS, cell surface markers were stained using the following antibodies: anti-CD3-PerCP-Cy5.5 (Clone: UCHT1, BD Biosciences), anti-CD8-Alexa Fluor 700 (Clone: RPA-T8, BioLegend Inc.), and Fixable Viability Dye eFluor 780 (Invitrogen Inc.). Data for the stained cells were acquired using a Symphony A3 instrument and analyzed using FlowJo software.

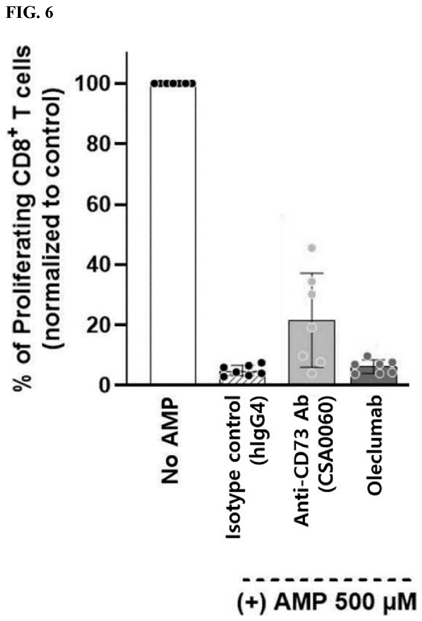

As a result, proliferation of CD8+ T cells was reduced following treatment with AMP and Vehicle (hIgG4). In contrast, treatment with the anti-CD73 antibody (CSA0060) increased CD8+ T cell proliferation. Meanwhile, treatment with Oleclumab alone did not show a tendency to increase CD8+ T cell proliferation (FIG. 6).

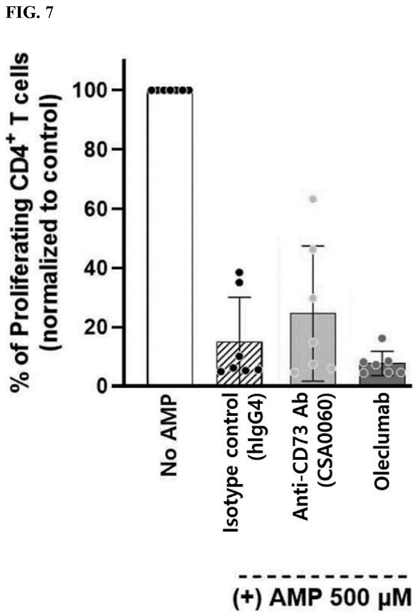

Furthermore, proliferation of CD4+ T cells was reduced following treatment with AMP and Vehicle (hIgG4). In contrast, treatment with the anti-CD73 antibody alone increased CD4+ T cell proliferation. Meanwhile, treatment with Oleclumab did not show a tendency to increase CD4+ T cell proliferation (FIG. 7).

Having described specific embodiments of the present invention in detail above, it is to be understood that variants and modifications thereof falling within the spirit of the invention may become apparent to those skilled in this art, and the scope of the present invention is to be determined by appended claims and their equivalents.

## Claims

1. An antibody or an antigen-binding fragment thereof that specifically binds to CD73 protein, comprising a heavy chain variable region comprising an HCDR1 having the amino acid sequence selected from the group consisting of SEQ ID NOs: 1 to 9 and 78 to 80; an HCDR2 having the amino acid sequence selected from the group consisting of SEQ ID NOs: 10 to 18 and 81 to 83; and an HCDR3 having the amino acid sequence selected from the group consisting of SEQ ID NOs: 19 to 28 and 84 to 86.

2. The antibody or the antigen-binding fragment thereof of claim 1, further comprising a light chain variable region comprising an LCDR1 having the amino acid sequence selected from the group consisting of SEQ ID NOs: 39 to 48; an LCDR2 having the amino acid sequence selected from the group consisting of SEQ ID NOs: 49 to 57; and LCDR3 having the amino acid sequence selected from the group consisting of SEQ ID NOs: 58 to 67.

3. The antibody or the antigen-binding fragment thereof of claim 2, wherein the antibody or the antigen-binding fragment thereof is selected from the group consisting of the following (a) to (j):
(a) an antibody or antigen-binding fragment thereof comprising a heavy chain variable region comprising HCDR1 having the amino acid sequence of SEQ ID NO: 1, HCDR2 having the amino acid sequence of SEQ ID NO: 10, and HCDR3 having the amino acid sequence of SEQ ID NO: 19; and a light chain variable region comprising LCDR1 having the amino acid sequence of SEQ ID NO: 39, LCDR2 having the amino acid sequence of SEQ ID NO: 49, and LCDR3 having the amino acid sequence of SEQ ID NO: 58;
(b) an antibody or antigen-binding fragment thereof comprising a heavy chain variable region comprising HCDR1 having the amino acid sequence of SEQ ID NO: 2, HCDR2 having the amino acid sequence of SEQ ID NO: 11, and HCDR3 having the amino acid sequence of SEQ ID NO: 20; and a light chain variable region comprising LCDR1 having the amino acid sequence of SEQ ID NO: 40, LCDR2 having the amino acid sequence of SEQ ID NO: 50, and LCDR3 having the amino acid sequence of SEQ ID NO: 59;
(c) an antibody or antigen-binding fragment thereof comprising a heavy chain variable region comprising HCDR1 having the amino acid sequence of SEQ ID NO: 3, HCDR2 having the amino acid sequence of SEQ ID NO: 12, and HCDR3 having the amino acid sequence of SEQ ID NO: 21; and a light chain variable region comprising LCDR1 having the amino acid sequence of SEQ ID NO: 41, LCDR2 having the amino acid sequence of SEQ ID NO: 51, and LCDR3 having the amino acid sequence of SEQ ID NO: 60;
(d) an antibody or antigen-binding fragment thereof comprising a heavy chain variable region comprising HCDR1 having the amino acid sequence of SEQ ID NO: 4, HCDR2 having the amino acid sequence of SEQ ID NO: 13, and HCDR3 having the amino acid sequence of SEQ ID NO: 22; and a light chain variable region comprising LCDR1 having the amino acid sequence of SEQ ID NO: 42, LCDR2 having the amino acid sequence of SEQ ID NO: 52, and LCDR3 having the amino acid sequence of SEQ ID NO: 61;
(e) an antibody or antigen-binding fragment thereof comprising a heavy chain variable region comprising HCDR1 having the amino acid sequence of SEQ ID NO: 5, HCDR2 having the amino acid sequence of SEQ ID NO: 14, and HCDR3 having the amino acid sequence of SEQ ID NO: 23; and a light chain variable region comprising LCDR1 having the amino acid sequence of SEQ ID NO: 43, LCDR2 having the amino acid sequence of SEQ ID NO: 53, and LCDR3 having the amino acid sequence of SEQ ID NO: 62;
(f) an antibody or antigen-binding fragment thereof comprising a heavy chain variable region comprising HCDR1 having the amino acid sequence of SEQ ID NO: 6, HCDR2 having the amino acid sequence of SEQ ID NO: 15, and HCDR3 having the amino acid sequence of SEQ ID NO: 24; and a light chain variable region comprising LCDR1 having the amino acid sequence of SEQ ID NO: 44, LCDR2 having the amino acid sequence of SEQ ID NO: 54, and LCDR3 having the amino acid sequence of SEQ ID NO: 63;
(g) an antibody or antigen-binding fragment thereof comprising a heavy chain variable region comprising HCDR1 having the amino acid sequence of SEQ ID NO: 7, HCDR2 having the amino acid sequence of SEQ ID NO: 16, and HCDR3 having the amino acid sequence of SEQ ID NO: 25; and a light chain variable region comprising LCDR1 having the amino acid sequence of SEQ ID NO: 45, LCDR2 having the amino acid sequence of SEQ ID NO: 49, and LCDR3 having the amino acid sequence of SEQ ID NO: 64;
(h) an antibody or antigen-binding fragment thereof comprising a heavy chain variable region comprising HCDR1 having the amino acid sequence of SEQ ID NO: 8, HCDR2 having the amino acid sequence of SEQ ID NO: 17, and HCDR3 having the amino acid sequence of SEQ ID NO: 26; and a light chain variable region comprising LCDR1 having the amino acid sequence of SEQ ID NO: 46, LCDR2 having the amino acid sequence of SEQ ID NO: 55, and LCDR3 having the amino acid sequence of SEQ ID NO: 65;
(i) an antibody or antigen-binding fragment thereof comprising a heavy chain variable region comprising HCDR1 having the amino acid sequence of SEQ ID NO: 3, HCDR2 having the amino acid sequence of SEQ ID NO: 12, and HCDR3 having the amino acid sequence of SEQ ID NO: 27; and a light chain variable region comprising LCDR1 having the amino acid sequence of SEQ ID NO: 47, LCDR2 having the amino acid sequence of SEQ ID NO: 56, and LCDR3 having the amino acid sequence of SEQ ID NO: 66; and
(j) an antibody or antigen-binding fragment thereof comprising a heavy chain variable region comprising HCDR1 having the amino acid sequence of SEQ ID NO: 9, HCDR2 having the amino acid sequence of SEQ ID NO: 18, and HCDR3 having the amino acid sequence of SEQ ID NO: 28; and a light chain variable region comprising LCDR1 having the amino acid sequence of SEQ ID NO: 48, LCDR2 having the amino acid sequence of SEQ ID NO: 57, and LCDR3 having the amino acid sequence of SEQ ID NO: 67.

4. The antibody or the antigen-binding fragment thereof of claim 3, wherein:
the (a) comprises a heavy chain variable region having the amino acid sequence of SEQ ID NO: 29 and a light chain variable region having the amino acid sequence of SEQ ID NO: 68;
the (b) comprises a heavy chain variable region having the amino acid sequence of SEQ ID NO: 30 and a light chain variable region having the amino acid sequence of SEQ ID NO: 69;
the (c) comprises a heavy chain variable region having the amino acid sequence of SEQ ID NO: 31 and a light chain variable region having the amino acid sequence of SEQ ID NO: 70;
the (d) comprises a heavy chain variable region having the amino acid sequence of SEQ ID NO: 32 and a light chain variable region having the amino acid sequence of SEQ ID NO: 71;
the (e) comprises a heavy chain variable region having the amino acid sequence of SEQ ID NO: 33 and a light chain variable region having the amino acid sequence of SEQ ID NO: 72;
the (f) comprises a heavy chain variable region having the amino acid sequence of SEQ ID NO: 34 and a light chain variable region having the amino acid sequence of SEQ ID NO: 73;
the (g) comprises a heavy chain variable region having the amino acid sequence of SEQ ID NO: 35 and a heavy chain variable region having the amino acid sequence of SEQ ID NO: 74;
the (h) comprises a heavy chain variable region having the amino acid sequence of SEQ ID NO: 36 and a light chain variable region having the amino acid sequence of SEQ ID NO: 75;
the (i) comprises a heavy chain variable region having the amino acid sequence of SEQ ID NO: 37 and a heavy chain variable region having the amino acid sequence of SEQ ID NO: 76; or
the (j) comprises a heavy chain variable region having the amino acid sequence of SEQ ID NO: 38 and a light chain variable region having the amino acid sequence of SEQ ID NO: 77.

5. The antibody or the antigen-binding fragment thereof of claim 1, wherein the antigen-binding fragment is a single-domain antibody.

6. The antibody or the antigen-binding fragment thereof of claim 5, wherein the single-domain antibody comprises an HCDR1 having the amino acid sequence selected from the group consisting of SEQ ID NOs: 78 to 80; an HCDR2 having the amino acid sequence region selected from the group consisting of SEQ ID NOs: 81 to 83; and an HCDR3 having the amino acid sequence selected from the group consisting of SEQ ID NOs: 84 to 86.

7. The antibody or the antigen-binding fragment thereof of claim 6, wherein the single-domain antibody comprises the amino acid sequence selected from the group consisting of SEQ ID NOs: 87 to 89.

8. A nucleic acid molecule encoding the antibody or the antigen-binding fragment thereof of claim 1.

9. A gene delivery system comprising the nucleic acid molecule of claim 8.

10. A host cell transformed with the gene delivery system of claim 9.

11. An antibody or an antigen-binding fragment thereof that specifically binds to a CD73 protein through one or more epitopes selected from the group consisting of:
(a) an epitope comprising the amino acid sequence of SEQ ID NO: 92;
(b) an epitope comprising the amino acid sequence of SEQ ID NO: 93; and
(c) an epitope comprising the amino acid sequence of SEQ ID NO: 94.

12. A pharmaceutical composition for preventing or treating cancer, comprising as an active ingredient the antibody or the antigen-binding fragment thereof of claim 1 or claim 11.

13. The pharmaceutical composition of claim 12, wherein the cancer is selected from the group consisting of leukemia, bladder cancer, brain tumor, melanoma, thyroid cancer, esophageal cancer, lung cancer, prostate cancer, breast cancer, colorectal cancer, gastric cancer, head and neck cancer, renal cancer, pancreatic cancer, liver cancer, neuroblastoma, retinoblastoma, salivary gland cancer, lymphoma, cervical cancer, ovarian cancer, and testicular cancer.
